# EUROPEAN PATENT APPLICATION

(11) **EP 4 527 405 A1**
(43) Date of publication of application: **26.03.2025**
(21) Application number: 22942800.8
(22) Date of filing: 16.05.2022
(51) Int. Cl.: A61K 38/44, A61K 38/16, A61P 27/02, A61P 31/00, A23L 33/195, A23K 20/189

(54) **SUPEROXIDE DISMUTASE AND USES THEREOF FOR PREVENTING OR TREATING DIABETIC RETINOPATHY OR UVEITIS**

(71) Applicant: Genofocus, Inc., Daejeon 34014 (KR); BiomLogic, Inc., Seoul 03923 (KR); Seoul National University Hospital, Seoul 03080 (KR)
(72) Inventor: PAN, Jae Gu, Seoul 03923 (KR); KIM, Eui Joong, Daejeon 34014 (KR); CHANG, In Ik, Seoul 03923 (KR); KIM, Jeong Hyun, Daejeon 34014 (KR); KIM, Jeong Hun, Seoul 06070 (KR)
(74) Representative: Ullrich & Naumann PartG mbB
(86) International application number: PCT/KR2022/007004
(87) International publication number: WO 2023/224136

(57) **Abstract**

The present invention relates to superoxide dismutase and uses thereof for preventing or treating diabetic retinopathy or uveitis. The superoxide dismutase according to the present invention can be effectively used in the prevention or treatment of diabetic retinopathy or uveitis, particularly through oral administration.

## Description

### Technical Field

The present disclosure relates to a use of a superoxide dismutase for prevention or treatment of diabetic retinopathy or uveitis. More specifically, the present disclosure relates to a use of a superoxide dismutase for prevention or treatment of diabetic retinopathy or uveitis, a composition, or a method.

### Background Art

Recently, there has been an increasing trend in vision loss due to various diseases such as retinal neurodegeneration due to aging. The eye, including the retina, is neural tissue related to visual function. Once the eye is damaged, recovery is difficult. Thus, it requires special attention. Diseases that cause vision loss include, for example, diabetic retinopathy, uveitis, cataracts, macular degeneration, retinal detachment, and the like.

Diabetic retinopathy is one of the chronic complications of diabetes and a major cause of blindness worldwide. It may lead to a delay in treatment, since vision deteriorates without noticeable pain. Diabetic retinopathy is treated through the common method of laser treatment, which prevents formation of new blood vessels and bleeding in the eye, thereby inhibiting rapid vision loss. However, in cases of diabetic macular edema, improvement of vision is difficult and there are limitations in applying laser treatment. To supplement this, a method of injecting steroids or vascular endothelial growth factor inhibitors (for example, dexamethasone) into the vitreous body is used. This has the advantage of inhibiting vascular endothelial cell growth factors and improving chronic inflammation in the retina of patients with diabetic retinopathy; however, there is a risk of side effects with long-term use.

Uveitis is an inflammatory disease of the uveal tract of the eye. Uveitis may resolve if it occurs as a transient condition. However, most cases of uveitis involve recurrent and persistent inflammation and are idiopathic with unknown causes. Therefore, uveitis may eventually cause serious visual impairment, leading to blindness. Uveitis is caused by infectious factors such as viruses, bacteria, fungi, and parasites, as well as non-infectious factors such as autoimmune diseases. Uveitis is predominantly an idiopathic endogenous disease for which no causative organism is identified, and it involves abnormalities in the immune system. Thus, corticosteroid preparations are primarily administered in the form of eye drops. In cases of acute inflammation, treatment involves topical application of steroid eye drops combined with cycloplegic eye drops. In cases of severe inflammation, treatment involves administration of systemic steroids; or in cases of chronic disease, treatment involves administration of immunosuppressants such as cyclosporine.

Conventional steroid preparations have a risk of side effects with long-term use, and cycloplegics have many disadvantages, such as causing photophobia and difficulty in reading close text. Therefore, there is a need to develop preparations that can prevent or treat eye diseases, in particular, diabetic retinopathy and/or uveitis.

### Disclosure of Invention

### Technical Problem

The object of the present disclosure is to solve all of the above-mentioned problems.

An object of the present disclosure is to provide a superoxide dismutase for preventing, ameliorating, or treating diabetic retinopathy or uveitis.

Another object of the present disclosure is to provide a superoxide dismutase, which is derived from *Bacillus amyloliquefaciens* and for which oral administration efficacy and safety are secured, for preventing, ameliorating or treating diabetic retinopathy or uveitis.

Yet another object of the present disclosure is to provide a superoxide dismutase, which is derived from Bacillus or *Bacillus amyloliquefaciens* and is secreted extracellularly for convenient production, for preventing or treating diabetic retinopathy or uveitis.

Still yet another object of the present disclosure is to provide a pharmaceutical composition or a therapeutic method for preventing or treating diabetic retinopathy or uveitis.

Still yet another object of the present disclosure is to provide a food or feed composition for preventing or ameliorating diabetic retinopathy or uveitis.

Still yet another object of the present disclosure is to provide a composition for oral administration, which comprises a superoxide dismutase, for preventing, ameliorating, or treating diabetic retinopathy or uveitis.

The object of the present disclosure is not limited to the above-mentioned objects. The objects of the present disclosure will become clearer from the following description and may be realized by means and combinations thereof as set forth in the claims.

### Solution to Problem

Representative configurations of the present disclosure to achieve the above-mentioned objects are as follows.

According to an aspect of the present disclosure, there is provided an improved superoxide dismutase (SOD) that exhibits an effect of preventing, ameliorating, or treating diabetic retinopathy or uveitis.

According to another aspect of the present disclosure, there is provided a superoxide dismutase, which is derived from a generally regarded as safe (GRAS) bacterium, such as Bacillus, for preventing, ameliorating, or treating diabetic retinopathy or uveitis.

According to yet another aspect of the present disclosure, there is provided a superoxide dismutase, which is derived from Bacillus or *Bacillus amyloliquefaciens* and is secreted extracellularly for convenient production, for preventing, ameliorating, or treating diabetic retinopathy or uveitis.

According to still yet another aspect of the present disclosure, there is provided an improved superoxide dismutase that exhibits an effect of preventing, ameliorating, or treating diabetic retinopathy or uveitis even in a case of being administered orally.

According to still yet another aspect of the present disclosure, there is provided a superoxide dismutase, which is derived from *Bacillus amyloliquefaciens* and for which oral administration efficacy and safety are secured, for preventing, ameliorating, or treating diabetic retinopathy or uveitis.

According to still yet another aspect of the present disclosure, there is provided a pharmaceutical composition for preventing or treating diabetic retinopathy or uveitis, comprising the superoxide dismutase as an active ingredient.

According to still yet another aspect of the present disclosure, there is provided a food or feed composition for preventing or ameliorating diabetic retinopathy or uveitis, comprising the superoxide dismutase as an active ingredient.

According to still yet another aspect of the present disclosure, there is provided an oral composition for preventing, ameliorating, or treating diabetic retinopathy or uveitis, comprising the superoxide dismutase as an active ingredient.

According to still yet another aspect of the present disclosure, there is provided a method for preventing, ameliorating, or treating diabetic retinopathy or uveitis, comprising administering to a subject the superoxide dismutase or the composition.

According to still yet another aspect of the present disclosure, there is provided a use of the superoxide dismutase for preventing, ameliorating, or treating diabetic retinopathy or uveitis.

According to still yet another aspect of the present disclosure, there is provided a use of the superoxide dismutase in manufacture of a medicament for prevention, amelioration, or treatment of diabetic retinopathy or uveitis.

### Advantageous Effects of Invention

The superoxide dismutase, composition, method, and kit according to the present disclosure can be effectively used for prevention or treatment of diabetic retinopathy or uveitis. In particular, it has been identified that the superoxide dismutase according to the present disclosure is effective in preventing or treating diabetic retinopathy or uveitis even in a case of being administered orally.

The superoxide dismutase according to the present disclosure is derived from Bacillus or *Bacillus amyloliquefaciens* which is a generally regarded as safe (GRAS) bacterium. This not only ensures oral administration efficacy and safety, but also provides production advantage of being directly recoverable from the supernatant during culture.

According to examples of the present disclosure, the SOD of the present disclosure was orally administered to diabetic mice and normal mice, and evaluation of retinal vascular leakage, evaluation of astrocyte immunofluorescence staining, and evaluation of retinal vascular staining were performed. The results showed that reduced retinal vascular permeability, recovery of astrocyte foot processes, inhibited pericyte loss, and inhibited acellular capillary formation were observed in the SOD-administered diabetic-induced mice. In addition, the SOD was orally administered to the mice with acute and chronic uveitis induced, and fundus evaluation and tissue evaluation were performed. As a result, it was confirmed that the inflammation was improved to a lower grade. Therefore, the superoxide dismutase according to the present disclosure can lead to reduced retinal vascular leakage and improved inflammatory condition, and thus can be effectively used to prevent, ameliorate, or treat diabetic retinopathy or uveitis.

### Brief Description of Drawings

FIG. 1 illustrates a schematic diagram for the double crossover recombination performed in Example 1.1.
FIG. 2 illustrates results obtained by identifying defective genes using PCR in the expression host GFBS220 and the production strain BSBA310. W represents wild-type B. *subtilis* KCTC 3135, 220 represents the expression host GFBS220, and 310 represents the SodA2 production strain BSBA310.
FIG. 3 illustrates comparison of the amino acid sequences between SodA (Mn-SOD identified through N-terminal sequencing from the culture supernatant of *Bacillus amyloliquesfaciens* strain GF423) and the superoxide dismutase (SodA2) of the present disclosure.
FIG. 4 illustrates an expression vector for overexpression of *sodA2* gene. Here, rrnB T1T2 represents a transcription terminator; rep(pBR322) represents a pBR322-derived replicon that operates in *E. coli.;* rep(pUB110) represents a pUB110-derived replicon that operates in *B. subtilis;* Kan^{R} represents a kanamycin resistance gene (aminoglycoside O-nucleotidyltransferase); and BJ27 promoter represents a strong promoter for *B. subtilis.*
FIG. 5 illustrates an overall procedure for preparing the production strain BSBA310.
FIGS. 6A and 6B illustrate results obtained by performing qualitative evaluation of retinal vascular leakage in the 3-week experimental groups with diabetic retinopathy (scale bar represents 500 µm), and FIG. 6C illustrates results obtained by performing quantitative evaluation thereof.
FIGS. 7A and 7B illustrate results obtained by performing qualitative evaluation of retinal vascular leakage in the 16-week experimental groups with diabetic retinopathy (scale bar represents 500 µm), and FIG. 7C illustrates results obtained by performing quantitative evaluation thereof.
FIGS. 8A and 8B illustrate results obtained by performing qualitative evaluation of astrocyte immunofluorescence staining in the 3-week experimental groups with diabetic retinopathy, and FIG. 8C illustrates results obtained by performing quantitative evaluation thereof.
FIGS. 9A and 9B illustrate results obtained by performing qualitative evaluation of astrocyte immunofluorescence staining in the 16-week experimental groups with diabetic retinopathy, and FIG. 9C illustrates results obtained by performing quantitative evaluation thereof.
FIGS. 10A and 10B illustrate results obtained by performing qualitative evaluation of retinal vascular staining in the 16-week experimental groups with diabetic retinopathy (scale bar represents 100 µm), and FIGS. 10C and 10D illustrate results obtained by performing quantitative evaluation thereof.
FIGS. 11A and 11B illustrate clinical score results obtained by performing uveitis fundus evaluation. FIG. 11A represents results obtained by performing fundus evaluation in the endotoxin-induced uveitis model, and FIG. 11B represents results obtained by performing fundus evaluation in the experimental autoimmune uveitis model.
FIG. 12 illustrates four regions of retina within the eye which are divided for evaluation of uveitis tissue.
FIGS. 13A to 13C illustrate results obtained by performing tissue evaluation of uveitis in an endotoxin-induced uveitis model. FIG. 13A shows results for the normal control group and the normal mouse group administered with SodA2, and FIG. 13B shows results for the endotoxin-induced uveitis model and the SodA2-administered model. The scale bars in FIGS. 13A and 13B represent 200 µm (left) and 100 µm (right), respectively. FIG. 13C shows results of grade distribution for the experimental groups.
FIGS. 14A to 14C illustrate obtained by performing tissue evaluation of uveitis in an experimental autoimmune uveitis model. FIG. 14A shows results for the normal mouse group and the normal mouse group administered with SodA2, and FIG. 14B shows results for the experimental autoimmune uveitis model and the SodA2-administered model. The scale bars in FIGS. 14A and 14B represent 200 µm (left) and 100 µm (right), respectively. FIG. 14C shows results of grade distribution for the experimental groups.

### Modes for Carrying out Invention

The detailed description of the present disclosure set forth below will be described with reference to specific drawings with respect to an embodiment in which the present disclosure may be practiced; however, the present disclosure is not limited thereto and, if properly described, is limited only by the appended claims, along with the full scope of equivalents to which such claims are entitled. It should be understood that various embodiments/examples of the present disclosure, although different, are not necessarily mutually exclusive. For example, a particular feature, structure, or characteristic described herein may be changed from one embodiment to another embodiment or implemented in combinations of embodiments without departing from the technical spirit and scope of the present disclosure. Unless otherwise indicated, terms used in describing the present disclosure are to be understood in their ordinary meaning and apply to the same terms used herein as well as to the aspect or embodiment of the disclosure for which the term is defined. For purposes of interpreting this specification, the following definitions will apply and whenever appropriate, terms used in the singular will also include the plural and vice versa.

### Definition

As used herein, the term "about" refers to a typical margin of error for each value known to those skill in the art.

As used herein, the term "subject" is used interchangeably with "patient," and may be any mammal in need of prevention or treatment of diabetic retinopathy or uveitis, such as primate (for example, human), companion animal (for example, dog, cat, and the like), domestic animal (for example, cow, pig, horse, sheep, goat, and the like), and laboratory animal (for example, rat, mouse, guinea pig, and the like). In an embodiment of the present disclosure, the subject is a human.

The term "treatment" includes prophylactic and/or therapeutic treatment. The prophylactic and/or therapeutic treatment includes all types of treatment recognized in the art and includes, for example, administering the pharmaceutical composition or SOD of the present disclosure to a subject. If it is administered prior to clinical manifestation of an unwanted or undesirable condition (for example, disease or other unwanted or undesirable state in the subject), the treatment is prophylactic treatment (for example, it protects the subject against developing the unwanted or undesirable condition). On the other hand, if it is administered after clinical manifestation of an unwanted or undesirable condition, the treatment is therapeutic treatment, such as to diminish, ameliorate, or stabilize the existing unwanted or undesirable condition or side effects thereof.

The meaning of the term "prevention" is well known in the art. When used in the context of a medical condition, such as diabetic retinopathy or uveitis, it means reducing frequency or delaying onset and symptoms of a medical condition (for example, discomfort, visual disorder, ocular vascular leakage, ocular surface damage, ocular surface inflammation, or the like) in a subject who has received the pharmaceutical composition or SOD of the present disclosure, as compared with a subject who did not receive the same.

The term "administration" refers to providing an active ingredient to a subject to achieve a prophylactic or therapeutic purpose (for example, prevention or treatment of diabetic retinopathy or uveitis).

### Diabetic retinopathy and uveitis

The present disclosure is based, at least in part, on the discovery that administration, in particular, oral administration of a superoxide dismutase (SOD) is effective in preventing or treating diabetic retinopathy or uveitis. Therefore, according to an aspect of the present disclosure, there is provided a use of an SOD for preventing or treating diabetic retinopathy or uveitis.

In an embodiment, diabetic retinopathy or uveitis may exhibit at least one symptom selected from the group consisting of vision impairment, ocular vascular leakage, and ocular inflammation.

The term "diabetic retinopathy" refers to an eye complication caused by sustained high blood glucose from diabetes, which leads to damage to the capillaries and results in vision impairment. Diabetic retinopathy initially has no symptoms, but as the macular area is affected, vision impairment occurs. Diabetic retinopathy is a disease that develops or progresses with various pathological features, such as microaneurysm, venous dilation, retinal hemorrhage, retinal infarction, macular edema, neovascularization, vitreous hemorrhage, and tractional membranes.

In an embodiment, the SOD may bring about at least one effect selected from reduced vascular permeability, recovery of astrocyte foot process, inhibited pericyte loss, and inhibited acellular capillary formation in diabetic retinopathy.

The term "uveitis" means inflammation of the inner part of the eye, in particular, inflammation of the middle layer (the uvea) of the eye. More specifically, uveitis includes anterior uveitis, which involves inflammation of the anterior parts of the uveal system, including inflammation of the iris (iritis) and inflammation of the iris and ciliary body (iridocyclitis); intermediate uveitis, which involves inflammation in the vitreous body; posterior uveitis, which involves inflammation of the parts of the uveal system behind the iris of the eye, including inflammation of the choroid (choroiditis) and inflammation of the choroid and retina (chorioretinitis), as well as panuveitis that affects the entire uveal system.

In an embodiment, the uveitis may include, but is not limited to, one or more selected from the group consisting of infectious factors and non-infectious factors. Specifically, the infectious factors of uveitis may comprise at least one selected from bacteria, viruses, fungi, and parasites, and the non-infectious factors may comprise at least one selected from autoimmune diseases, tumor, trauma, surgery, and systemic diseases.

In an embodiment, the SOD may exhibit an improvement or alleviation effect in at least one fundus evaluation indicator selected from focal lesion, linear lesion, chorioretinal lesion, confluent lesion, vasculitis, vitritis, vitreous hemorrhage, papilledema, and retinal detachment; an improvement or alleviation effect in at least one tissue evaluation indicator selected from inflammatory cell infiltration, retinal fold, intra/subretinal hemorrhage, intra/subretinal exudate, and retinal damage; or both of these effects.

### Superoxide dismutase (SOD)

According to another aspect of the present disclosure, there is provided an improved superoxide dismutase (SOD) that has an effect of preventing, ameliorating, or treating diabetic retinopathy or uveitis.

A superoxide dismutase (SOD) is an enzyme that alternately catalyzes dismutation of superoxide (O₂ ^{• -}) radical into either ordinary molecular oxygen (O₂) or hydrogen peroxide (H₂O₂). SODs play a key role in decreasing oxidative stress by removing reactive oxygen species. SODs are widely distributed in prokaryotic and eukaryotic cells and are classified into four classes based on their different types of metal centers (copper/zinc, nickel, manganese, and iron). Manganese-containing SODs (Mn-SODs) are widely present in many bacteria, chloroplasts, mitochondria, and cytosol of eukaryotic cells. The term "SOD" may be used interchangeably with a polypeptide having superoxide dismutase activity.

In an embodiment, the SOD may bind to manganese (Mn-SOD). Specifically, the SOD may be a deamidated Mn-SOD. More specifically, amino acid residues 73 and 136 of the SOD may be substituted with Asp residues, with respect to SEQ ID NO: 2. Even more specifically, the SOD may comprise or consist of the amino acid sequence represented by SEQ ID NO: 4.

The SOD or polypeptide having SOD activity of the present disclosure is interpreted to include amino acid sequences showing substantial identity to the above-mentioned amino acid sequence. The substantial identity means that in a case where aligned amino acid sequences are analyzed using an algorithm commonly used in the art, the sequences show sequence identity of 80% or higher, preferably 90% or higher, more preferably 95% or higher, and most preferably 98% or higher.

In another embodiment, the SOD may be a modified or engineered polypeptide having SOD enzymatic activity, and the polypeptide may comprise one or more mutations, for example, deletion, insertion, or substitution of one or more amino acids, which may or may not affect various aspects (in vivo, in vitro, or ex vivo stability, homogeneity, and/or conformational change). In addition, the polypeptide may further comprise a heterologous substance (for example, a tag known in the art, including HIS tag, HA tag, and myc tag, GFC, and/or Fc domain of an antibody) for purification, detection, or increased stability.

In an embodiment, the SOD may be derived from natural or recombinant microorganisms, and may be an enzyme produced through a process of isolation or purification from various sources.

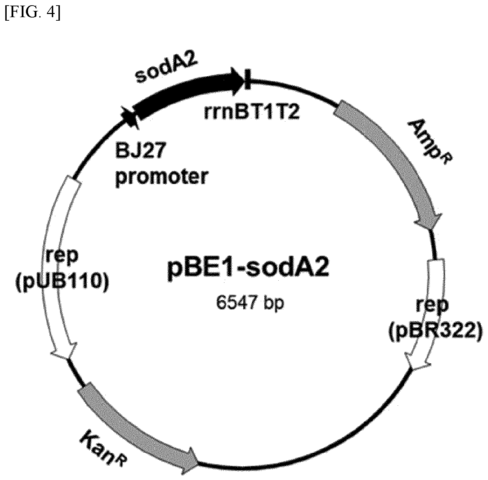

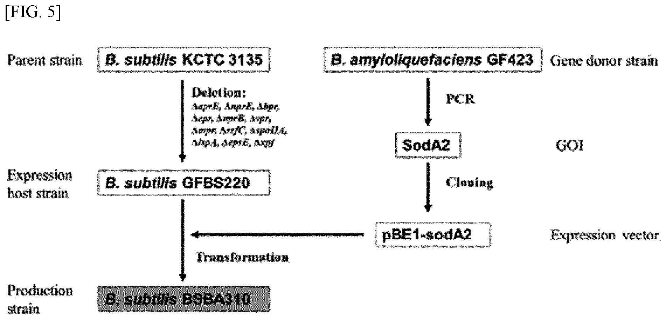

In an embodiment, the SOD may be derived from natural or recombinant microorganisms. For example, the SOD may be derived from bacteria. Preferably, the SOD may be derived from bacteria that are generally regarded as safe (GRAS) for use in drugs or foods. Specifically, the SOD may be derived from a Bacillus species strain. More specifically, the SOD may be derived from a *Bacillus amyloliquefaciens* strain. For example, the SOD may be derived from the *Bacillus amyloliquefaciens* strain GF423 (KCTC 13222 BP). The GF423 (KCTC 13222 BP) strain was deposited with the Korea Research Institute of Bioscience and Biotechnology on March 6, 2017. In addition, the SOD may be derived from a recombinant strain comprising the expression vector shown in FIG. 4. In addition, the SOD may be derived from a recombinant strain produced by a method including the method shown in FIG. 5. The recombinant strain may be a Bacillus species strain that comprises a nucleotide sequence encoding a polypeptide that comprises the amino acid sequence of SEQ ID NO: 4, in which the following genes are deleted: *AprE, NprE, Bpr, Epr, NprB, Vpr, Mpr, IspA, SrfAC, spoIIAc, EpsE,* and *Xpf.* For detailed procedures for preparing the strain, see Example 1.

From the viewpoint that the SOD derived from the above-mentioned strain is an enzyme secreted extracellularly, in a case where an SOD is produced using the strain, it is possible to mass-produce an SOD, which is safe for humans, without going through an expensive purification process (for example, column purification), and this enables efficient production.

In an embodiment, the SOD may be isolated or purified from a variety of sources, including natural or recombinant hosts. For example, the SOD having activity of preventing or treating diabetic retinopathy or uveitis may be extracted from a culture supernatant of the *Bacillus amyloliquefaciens* strain GF423. Briefly, the Bacillus *amyloliquefaciens* strain GF423 may be first cultured in various types of media to obtain a culture. For example, the strain is grown at about 25°C to about 42°C for 1 day to 4 days using a complex medium (pH 6.0 to 7.0). Other suitable media for culturing the *Bacillus amyloliquefaciens* strain GF423 include Luria-Bertani (LB) medium, International Streptomyces Project (ISP) medium, nutrient agar (NA) medium, brain heart infusion agar (BHI) medium, sabouraud dextrose agar (SDA) medium, potato dextrose agar (PDA) medium, nutrient broth (NB) medium, and the like. Preferably, LB medium, ISP medium, BHI medium, SDA medium, or NB medium may be used. In addition, the SOD may be obtained from other natural or recombinant hosts using information provided in databases such as PubMed or BRENDA (www.brenda-enzymes.org).

In an embodiment, the SOD may be an isolated or purified enzyme. Here, the isolated or purified SOD, or a biologically active portion thereof is substantially free of cellular material or other contaminating proteins from the cell or tissue source from which it is derived. For example, the purified product may be separated purely from a strain culture through ultrafiltration, ammonium sulfate treatment, column purification, concentration, or the like, or may be a culture concentrate obtained therefrom through ultrafiltration, concentration, or the like. The phrase "substantially free of cellular material" includes preparations of a protein obtained by separating the protein from cellular components of the cell from which the protein is isolated or recombinantly produced. Specifically, the phrase "substantially free of cellular material" may include preparations of a protein in which unwanted proteins are present in an amount of less than about 30%, preferably less than about 20%, more preferably less than about 10%, and most preferably less than about 5% by dry weight.

In another embodiment, the SOD may be preferably purified by the following purification method, but the method is not limited thereto. The culture obtained by culturing the *Bacillus amyloliquefaciens* strain GF423 is centrifuged to collect a culture supernatant. The supernatant fraction is pretreated by solid-phase extraction, and then isolated and purified by chromatography. Here, various modes of chromatography may be used to purify the SOD. Preferably, hydrophobic interaction chromatography is used.

In an embodiment, the SOD may be included in a form of a strain lysate, a strain culture, a strain culture concentrate, or a strain culture extract, or a dried form thereof. Here, the "strain lysate" may be obtained by culturing a strain and disrupting it mechanically or chemically, and may include any material obtained therefrom after additional processes such as extraction, dilution, concentration, purification, and the like. The "strain culture" may refer to a culture itself obtained by culturing a strain or a supernatant thereof. The "strain culture concentrate" refers to a product that is separated purely from a strain culture through ultrafiltration, ammonium sulfate treatment, column purification, concentration, or the like, or a culture concentrate obtained therefrom through ultrafiltration, concentration, or the like. The "strain culture extract" refers to a product that is extracted from the culture or a concentrate thereof, and may include an extract, a diluted or concentrated solution of the extract, a dried product obtained by drying the extract, or a crude or purified product thereof, or a fraction obtained by fractionating the same. The dried form may include a lyophilized form.

In an example, it was identified that in a case of being administered orally to a subject in need of prevention or treatment of diabetic retinopathy, the SOD exhibits at least one effect of reduced vascular permeability, recovery of astrocyte foot process, inhibited pericyte loss, and inhibited acellular capillary formation.

In another example, in a case of being administered orally to a subject in need of prevention or treatment of uveitis, the SOD exhibits an improvement or alleviation effect in at least one fundus evaluation indicator selected from focal lesion, linear lesion, chorioretinal lesion, confluent lesion, vasculitis, vitritis, vitreous hemorrhage, papilledema, and retinal detachment; an improvement or alleviation effect in at least one tissue evaluation indicator selected from inflammatory cell infiltration, retinal fold, intra/subretinal hemorrhage, intra/subretinal exudate, and retinal damage; or both of these effects.

### Pharmaceutical composition

According to yet another aspect of the present disclosure, there is provided a pharmaceutical composition for preventing or treating diabetic retinopathy or uveitis, comprising a superoxide dismutase (SOD) as an active ingredient.

In an embodiment, the SOD according to the present disclosure may be combined with a pharmaceutically acceptable carrier, excipient and/or diluent to form a pharmaceutical composition for prevention or treatment of diabetic retinopathy or uveitis. The pharmaceutical composition of the present disclosure may be used interchangeably with the term veterinary composition when applied to an animal other than a human.

The pharmaceutical or veterinary composition of the present disclosure may further comprise one or more selected from the group consisting of pharmaceutically acceptable carriers, excipients, and diluents. The pharmaceutically acceptable carrier, excipient, and/or diluent may be those commonly used in the art. The carrier, excipient, or diluent may include, for example, lactose, dextrose, sucrose, sorbitol, mannitol, xylitol, erythritol, maltitol, starch, gum acacia, alginate, gelatin, calcium phosphate, calcium silicate, cellulose, methyl cellulose, hydroxypropyl methyl cellulose, microcrystalline cellulose, polyvinyl pyrrolidone, water, methyl hydroxybenzoate, propyl hydroxybenzoate, talc, magnesium stearate, silicon dioxide, and mineral oil, but are not limited thereto.

In a case of performing preparation, the preparation may be achieved by using an additive such as a filler, an extender, a binder, a wetting agent, a disintegrant, or a surfactant. The additive for preparation may be appropriately selected from those commonly used in the pharmaceutical field.

In addition, the pharmaceutical or veterinary composition of the present disclosure may be formulated into a desired form depending on the method of use, and in particular, the formulation may be achieved by employing a method known in the art to provide rapid, sustained, or delayed release of the active ingredient after being administered to a mammal. Specific examples of such a formulation include tablet, pill, powder, granule, syrup, solution, capsule, suspension, emulsion, injection solution, plaster, lotion, liniment, lemonade, aerosol, extract, elixir, ointment, fluid extract, needle, cream, soft or hard gelatin capsule, patch, and the like.

Furthermore, the pharmaceutical or veterinary composition of the present disclosure may preferably be formulated using an appropriate method known in the art or a method disclosed in Remington's Pharmaceutical Science (latest edition), Mack Publishing Company, Easton PA.

In an embodiment, the SOD may be administered orally or parenterally. In a case of being orally administered, the SOD may be coated with shellac for protection from gastric acid, but the coating agent is not limited thereto. Examples of the coating agent suitable for use in the present disclosure include shellac, ethyl cellulose, hydroxypropyl methylcellulose, hydroxypropyl methylcellulose phthalate, zein, Eudragit, and combinations thereof. In a case where the SOD is coated, the SOD may be coated in solution. Specifically, a purified solution and a shellac-containing solution are mixed and then lyophilized. This lyophilized sample may be made into powder and stored at about 4°C until use. Solid preparations for oral administration include tablets, pills, powders, granules, capsules and the like, and these solid preparations may be prepared by mixing a complex composition with at least one excipient, such as starch, calcium carbonate, sucrose, lactose, gelatin and the like. In addition to simple excipients, a lubricant such as magnesium stearate and talc may also be used. Examples of liquid preparations for oral administration include suspensions, oral solutions, emulsions, and syrups. In addition to commonly used simple diluents such as water and liquid paraffin, various types of excipients, such as a wetting agent, a sweetener, a fragrance, and a preservative, may be used.

For parenteral administration, sublingual administration, intraocular administration including intravitreal administration, nasal spray, topical application to the skin, patch, intraperitoneal injection, rectal injection, subcutaneous injection, intravenous injection, intramuscular injection, or intrathoracic injection may be selected.

In addition, the pharmaceutical or veterinary composition of the present disclosure is administered in a pharmaceutically or veterinary effective amount. The term "pharmaceutically effective amount" or "veterinary effective amount" refers to an amount sufficient to treat a disease at a reasonable benefit/risk ratio applicable to any medical or veterinary treatment. A level of the effective amount may be determined depending on factors including the patient's or animal's body weight, gender, age, health status, severity of disease, activity of the drug, sensitivity to the drug, administration time, administration route and excretion rate, duration of treatment, and drugs used concurrently therewith, and other factors well known in the medical field. Preferably, the pharmaceutical or veterinary composition of the present disclosure may comprise an SOD in an amount of about 2 to about 1,500 U/mg, specifically about 5 to about 1,000 U/mg, more specifically about 10 to about 800 U/mg, and even more specifically about 100 to about 500 U/mg, based on the total weight of the composition.

The pharmaceutical or veterinary composition of the present disclosure may be administered as an individual therapeutic agent or in combination with other therapeutic agents, in which the administration may be carried out in a sequential or simultaneous manner. In addition, the pharmaceutical composition may be administered once or multiple times as needed. Taking all of the above factors into consideration, it is important to administer a minimum amount that allows the maximum effect to be obtained with no adverse effects, and such an amount may be easily determined by those skilled in the art.

### Therapeutic or prophylactic method

According to still yet another aspect of the present disclosure, there is provided a method for treating or preventing diabetic retinopathy or uveitis, comprising administering, to a subject having or at risk of diabetic retinopathy or uveitis, the SOD or pharmaceutical composition according to the present disclosure.

The term "administration" includes any of a variety of administrations that accomplish a desired action and enable treatment. The route of administration may be oral, parenteral, inhalation, topical, or local administration (for example, intralesional administration). For example, parenteral administration may include, but is not limited to, intravenous, subcutaneous, intraperitoneal, intraarterial, and intraocular administration. In a case of oral administration, as described above, SOD may be administered in a form coated with a coating agent such as shellac.

An effective amount or effective non-toxic amount of the SOD according to the present disclosure may be determined by conventional experimentation. For example, a therapeutically active amount of the SOD of the present disclosure may vary depending on factors such as disease stage, disease severity, subject's age, gender, medical complications, and body weight. Dosage and dosing regimen of the SOD of the present disclosure may be adjusted to provide an optimal therapeutic response. For example, several divided doses may be administered daily, weekly, every two weeks, every three weeks, every four weeks and the like, and/or the dose may be proportionally reduced or increased depending on exigencies of therapeutic situation.

In an embodiment, the method may comprise administering one or more additional agents for treating or preventing diabetic retinopathy or uveitis. The additional agents include, but are not limited to, any agent that can improve or ameliorate diabetic retinopathy or uveitis, such as compounds, gene therapy agents, proteins (including antibodies), and the like. The one or more additional agents may be administered simultaneously with, sequentially, or in reverse order to the SOD or pharmaceutical composition. In addition, the individual agents may be administered to a subject by the same or different routes.

### Food composition

According to still yet another aspect of the present disclosure, there is provided a food composition for ameliorating or preventing diabetic retinopathy or uveitis, comprising the SOD according to the present disclosure as an active ingredient. Such a food composition includes a medical or nutraceutical food composition.

The term "medical food" or "nutraceutical food" refers to a food manufactured from raw materials or ingredients that are likely to have beneficial functions in the human body, the food maintaining normal function or activating physiological functions of the human body to maintain or improve health. This food is defined by the Ministry of Food and Drug Safety, but is not limited thereto. The food does not exclude any common health food from its meaning.

In addition, the food includes, but is not limited to, various foods, food additives, beverages (for example, functional drinks, natural fruit juices, and vegetable drinks), gum, tea, vitamin complexes, health functional foods, other functional foods, and the like.

The food may be manufactured by conventional methods known in the art. For example, the medical food, nutraceutical food, or health functional food may be formulated into one selected from the group consisting of tablet, pill, powder, granule, capsule, and liquid formulation by further comprising at least one of a carrier, a diluent, an excipient, and an additive, in addition to the SOD, for the purpose of preventing or ameliorating diabetic retinopathy or uveitis. Specific examples of the carrier, excipient, diluent, and additive are well known in the art, and those skilled in the art are capable of combining appropriate ingredients depending on the formulation to manufacture the food.

An amount of the superoxide dismutase according to the present disclosure as an active ingredient in the above-described formulation may be adjusted appropriately depending on the form and purpose of use, the patient's condition, type and severity of symptoms, and the like. The amount may be, but is not limited to, 0.001 to 99.9% by weight, and preferably 0.01 to 50% by weight, based on the weight of solid content.

A dose of the food of the present disclosure may vary depending on a patient's age, body weight, gender, dosage form, health status, and severity of disease, and the administration may be done once a day or in divided doses several times a day at regular time intervals depending on the judgment of a doctor or pharmacist. For example, the daily dosage may be 10 to 1,000 mg/kg based on the active ingredient content. The dosage is an example of an average case and may increase or decrease depending on individual differences. In a case where the daily dosage of the health functional food of the present disclosure is less than the above-mentioned dosage, it is not possible to obtain significant effects. In a case where the daily dosage exceeds the above-mentioned dosage, it is not only uneconomical but also undesirable side effects may occur because such a dosage is outside the usual dosage range.

### Feed composition

According to still yet another aspect of the present disclosure, there is provided a feed composition for preventing or ameliorating diabetic retinopathy or uveitis, comprising the SOD according to the present disclosure as an active ingredient. In the present disclosure, the food composition may include a feed composition. This feed composition of the present disclosure may be prepared in any formulation commonly used in the art. For example, the feed composition of the present disclosure may further comprise an auxiliary ingredient such as amino acids, inorganic salts, vitamins, antibiotics, antibacterial substances, antioxidants, antifungal enzymes, and other microbial agents in the form of live bacteria; grain such as ground or broken wheat, oats, barley, corn, and rice; vegetable protein feed such as those comprising rapeseed, soybean, and sunflower as main ingredients; animal protein feed such as blood meal, meat meal, bone meal, and fish meal; a dry ingredient consisting of sugar and a dairy product such as various powdered milk and whey powder; lipid such as animal fat and vegetable fat, optionally liquefied by heating; and an additive such as nutritional supplements, digestion and absorption enhancers, growth promoters, and disease prevention agents.

The feed composition of the present disclosure may be in a form of a powder or liquid preparation, and may comprise an excipient that is added to feed (calcium carbonate, wheat shorts, zeolite, corn meal, rice bran, or the like).

### Kit

The composition of the present disclosure may be provided in a form of a kit. For example, the kit may comprise the composition of the present disclosure (for example, a modified polypeptide, a pharmaceutical composition, a food composition, a feed composition, a composition for combined administration comprising at least one additional agent, or a combination thereof) packaged in a suitable container, and may further comprise instructions for use thereof. In addition, the kit may also comprise components such as an administration tool packaged in a separate container.

Hereinafter, the present disclosure will be described in more detail by way of the following examples. The following examples are presented to help understand the present disclosure. These examples are not intended to limit a scope of the present disclosure in any way and should not be construed as limiting the present disclosure.

### Examples

### Example 1. Preparation of recombinant strain expressing superoxide dismutase (SodA2)

### Example 1.1. Preparation of expression host GFBS220

The expression host GFBS220 was prepared using *Bacillus subtilis* KCTC 3135. *B. subtilis* KCTC 3135 was obtained from the Korea Research Institute of Bioscience and Biotechnology, Biological Resources Center (KCTC). To facilitate post-processing, the genes shown in Table 1 below were removed from the genome of *B. subtilis* KCTC 3135.

**[Table 1]**

| Gene name | Protein name | Function |
|---|---|---|
| *AprE* | Serine alkaline protease (subtilisin E) | Extracellular protease |
| *NprE* | Extracellular neutral metalloprotease | Extracellular protease |
| *Bpr* | Bacillopeptidase F | Extracellular protease |
| *Epr* | Extracellular serine protease | Extracellular protease |
| *NprB* | Extracellular neutral protease B | Extracellular protease |
| *Vpr* | Extracellular serine protease | Extracellular protease |
| *Mpr* | Extracellular metalloprotease | Extracellular protease |
| *IspA* | Intracellular serine protease | Intracellular protease |
| *SrfAC* | Surfactin synthase | Surfactin synthesis |
| *spoIIAC* | RNA polymerase sporulation-specific sigma factor (sigma-F) | Sporulation |
| *EpsE* | Putative glycosyltransferase | Extracellular polysaccharide |
| *Xpf* | RNA polymerase sigma factor | Transcription of PBSX prophage gene |

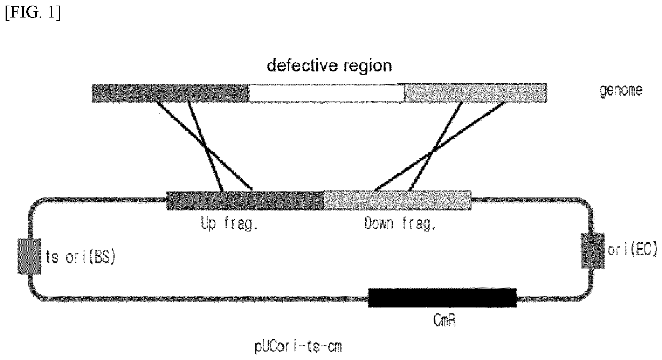

Removal of the genes was performed using double crossover recombination on the genome (FIG. 1). Specifically, DNA segments (up frag. and down frag.), which have homology to the DNA base sequences flanking the gene to be manipulated, were cloned into the vector pUCori-ts-cm that has a temperature-sensitive replication origin. PCR conditions and primers used for cloning are shown in Tables 2 and 3, respectively.

**[Table 2]**

| **PCR steps** | **Temperature** | **Time** |
|---|---|---|
| First denaturation | 95°C | 30 s |
| 30 cycles | 95°C | 30 s |
| | 50°C | 30 s |
| | 72°C | 1 min/kb |
| Final elongation | 72°C | 10 min |
| DNA polymerase used in PCR is *Taq* DNA polymerase (NEB, USA). | | |

**[Table 3]**

| Target gene | Homologous | | Primer sequence (5'→3') | SEQ ID NO |
|---|---|---|---|---|
| | pair | | | |
| AprE | fragment 1 | F | ctacggggtctgacgcagatatcgtaaccgaagaacg | 5 |
| | | R | tgttgcagacatgttgctgaacgccatc | 6 |
| | fragment 2 | F | caacatgtctgcaacatatcttggaaac | 7 |
| | | R | gataagctgtcaaaccagagattggataggctatcaag | 8 |
| NprE | fragment 1 | F | ctacggggtctgacgcagggcattactgcaccataatc | 9 |
| | | R | gaggtacgccttcagcagcctgaacacc | 10 |
| | fragment 2 | F | gctgaaggcgtacctcacgccttcttcc | 11 |
| | | R | gataagctgtcaaaccagttgtcagatttgcatccttc | 12 |
| Bpr | fragment 1 | F | ctacggggtctgacgcagagcaatttccagcccgcttc | 13 |
| | | R | gtatgatgaggcatgaacagcaatcccg | 14 |
| | fragment 2 | F | tcatgcctcatcatactcaacaagggtg | 15 |
| | | R | gataagctgtcaaaccagttcccagccggatgttcaac | 16 |
| Epr | fragment 1 | F | ctacggggtctgacgcaggctttctgccagccgatagg | 17 |
| | | R | tgtgtcaaagccgttatgcttggctccg | 18 |
| | fragment 2 | F | taacggctttgacacagcacaaactgcc | 19 |
| | | R | gataagctgtcaaaccaggtctcccatacatgaacgac | 20 |
| NprB | fragment 1 | F | ctacggggtctgacgcagagtaatcttgtaggaggctg | 21 |
| | | R | tgtgactgtcatattcaccgtcgtgtgg | 22 |
| | fragment 2 | F | tgaatatgacagtcacacagtattccgcc | 23 |
| | | R | gataagctgtcaaaccagattaggatacggtctggctg | 24 |
| Vpr | fragment 1 | F | ctacggggtctgacgcagatgtacgctgagccgaatag | 25 |
| | | R | ggaatcacatttgcggagatacggcgtc | 26 |
| | fragment 2 | F | ccgcaaatgtgattccggcacatcaaac | 27 |
| | | R | gataagctgtcaaaccagaccgttttccgaatctgacc | 28 |
| Mpr | fragment 1 | F | ctacggggtctgacgcagcgttatcccgaatgcccgtg | 29 |
| | | R | ggctttgttcctttagtgtcaaccaccc | 30 |
| | fragment 2 | F | ctaaaggaacaaagccgattcgctccgc | 31 |
| | | R | gataagctgtcaaaccagaaattgactgctccacgctg | 32 |
| SrfAC | fragment 1 | F | ctacggggtctgacgcaggatatgtattacctatcgcc | 33 |
| | | R | gccccttcatccagttcactgcttccttg | 34 |
| | fragment 2 | F | ggaagcagtgaactggatgaaggggcttcgctg | 35 |
| | | R | gataagctgtcaaaccagaacgcttcgacatcactttc | 36 |
| spoIIA | fragment 1 | F | ctacggggtctgacgcagagcggacgatgggagactgg | 37 |
| | | R | gccacctcatgcagccgatctggaagag | 38 |
| | fragment 2 | F | ggctgcatgaggtggctgagcggctcgg | 39 |
| | | R | gataagctgtcaaaccagctcattgttttggtgagctg | 40 |
| IspA | fragment 1 | F | ctacggggtctgacgcagtgctgcttggcattcatttc | 41 |
| | | R | gccattgaagcaatgcctccgtttgaatc | 42 |
| | fragment 2 | F | acggaggcattgcttcaatggctgcccctcatg | 43 |
| | | R | gataagctgtcaaaccagtcaatgctctcgtcatattc | 44 |
| EpsE | fragment 1 | F | ctacggggtctgacgcagcaagcaaatgggctgggagg | 45 |
| | | R | gacaagccatgctttctgccaaagtgcg | 46 |
| | fragment 2 | F | gaaagcatggcttgtcaagcatgaatag | 47 |
| | | R | gataagctgtcaaaccagtgttgtatacattcagcagc | 48 |
| Xpf | fragment 1 | F | gatcctctacggttcatccatgtccgaac | 49 |
| | | R | tctatgatcctcctcatttttg | 50 |
| | fragment 2 | F | gaggaggatcatagaaagcttgtcatacgtttgcc | 51 |
| | | R | gagttttcgttcggatcctccgctttttgtttg | 52 |

The thus produced vectors were introduced into *B. subtilis* cells, and then transformants (single crossover homologous recombination) were selected using medium containing chloramphenicol at 37°C (non-replication temperature). The selected transformants were cultured (second crossover) at 30°C (replication-permissive temperature) in LB medium without antibiotics, and then cultured at 39°C in LB agar medium without antibiotics. Colonies susceptible to chloramphenicol were selected from the resulting colonies (plasmid curing). The selected colonies were subjected to PCR to obtain double crossover recombinants, and second pure separation was performed to select the final recombinant, which was named GFBS220.

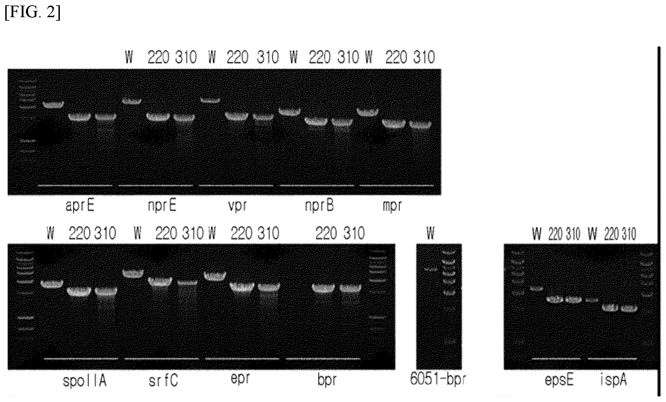

The defective gene regions of GFBS220 were amplified with the primers shown in Table 4 and PCR, and then identified using agarose gel electrophoresis. The results obtained by identifying the defective genes using PCR in the expression host GFBS220 are shown in FIG. 2.

**[Table 4]**

| Gene region | PCR primer (SEQ ID NO) | Size of PCR product of I | Size of PCR product |
|---|---|---|---|
| (locus) | | *B. subtilis* KCTC 3135 | of GFBSL210 |
| *AprE* | F caaggcttgaaataacgttg (SEQ ID NO: 53) | 3115bp | 2116bp |
| | R ttcgctgattacaacattgg (SEQ ID NO: 54) | | |
| *NprE* | F ggaacagatgatagctcatc (SEQ ID NO: 55) | 3487bp | 2145bp |
| | R acactccgagaaggctgaag (SEQ ID NO: 56) | | |
| *Vpr* | F ctgcggcctgcacagccgcc (SEQ ID NO: 57) | 3733bp | 2293bp |
| | R cgctgaatgacggtggtaag (SEQ ID NO: 58) | | |
| *NprB* | F agtaatcttgtaggaggctg (SEQ ID NO: 59) | 2666bp | 2153bp |
| | R cgccaatgaagtgaaggagg (SEQ ID NO: 60) | | |
| *Mpr* | F ggttgaggcgattgcgacgg (SEQ ID NO: 61) | 2734bp | 2076bp |
| | R ccattctgcaaaatcagctc (SEQ ID NO: 62) | | |
| *spoIIAC* | F aagatatgaagaaagccggg (SEQ ID NO: 63) | 2628bp | 2102bp |
| | R acagccgcttcataagcctg (SEQ ID NO: 64) | | |
| *ThrC* | F tcaagctgtcatgtacggag (SEQ ID NO: 65) | 376bp | 5455bp |
| | R tccgatacgaatggctgtcg (SEQ ID NO: 66) | | |

### Example 1.2. Construction of expression vector pBE1-sodA2

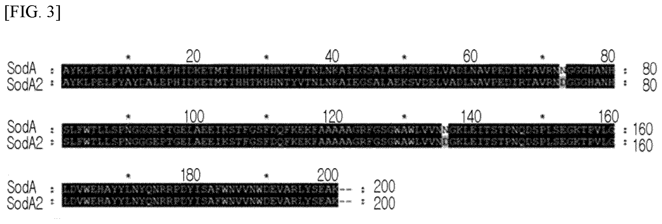

An Mn-SOD was identified in the culture supernatant of *Bacillus amyloliquefaciens* strain GF423 through N-terminal sequencing (Kang et al., 2018). The GF423 strain was deposited with the Korea Research Institute of Bioscience and Biotechnology on March 6, 2017 (under accession number of KCTC 13222 BP). The gene of the Mn-SOD was named *sodA* according to the nomenclature for bacterial SOD. For the amino acid sequence thereof, see FIG. 3 and SEQ ID NO: 2 (for the nucleotide sequence thereof, see SEQ ID NO: 1). LC-UV-MS/MS peptide mapping of the enzyme preparation produced 100% amino acid sequence coverage; however, deamidation was observed at Asn73 and Asn136 residues at 73% and 17% abundance, respectively. Therefore, to improve homogeneity of the purified enzyme, both Asn residues were replaced with Asp residues. For the amino acid sequence of such variant sodA, named SodA2, see FIG. 3 and SEQ ID NO: 4 (for the nucleotide sequence thereof, see SEQ ID NO: 3).

The *sodA2* gene was amplified with duplicate elongation PCR using four primers (Table 5) and the genome of *Bacillus amyloliquefaciens* GF423 as a template. Nucleotides for replacement of Asn with Asp are underlined.

**[Table 5]**

| Primer | | Sequence (5'→3') | SEQ ID NO |
|---|---|---|---|
| 1 | SOD F | GGAGGAATTACAATGGCTTACAA | 45 |
| 2 | D74 R | TGCATGTCCGCCGCCATCGTTGCGGAC | 46 |
| 3 | D74 F | CAGTCCGCAACGATGGCGGCGGACATG | 47 |
| 4 | D137 R | TTCAAGTTTGCCGTCGTTTACAACGAGC | 48 |
| 5 | D137 F | CTCGTTGTAAACGACGGCAAACTTG | 49 |
| 6 | SOD R | CAAAACAGAAGCTTCATTATTTTGCT | 50 |

The amplified DNA fragment containing the *sodA2* gene and the plasmid pBE1 linearized by treatment with *Nde*I and *Hind*III were assembled *in vitro* by the SLIC method (Jeong et al. 2012), and then the resulting construct was transformed into *E. coli* C2984H. Correct clones were screened by restriction enzyme mapping and identified by nucleotide sequencing. The resulting expression vector pBE1-sodA2 is shown in FIG. 4.

### Example 1.3. Preparation of production strain BSBA310

The expression vector pBE1-sodA2 was transformed into *B. subtilis* GFBS220. Strains were selected from the transformants, and pure clones were established by two single colony isolation procedures in LB2 medium. The selected strain was named BSBA310 and stored at -80°C using the glycerol stock method. The results obtained by identifying the defective genes using PCR in this production strain BSBA310 are shown in FIG. 2. In addition, the overall procedure for preparing the production strain BSBA310 is summarized in FIG. 5.

### Example 2. Isolation and purification of superoxide dismutase (SodA2) from production strain BSBA310

### Example 2.1. Culture of production strain BSBA310

For culture of the production strain BSBA310 obtained in Example 1, the single colony formed on LB agar medium (LB (Luria-Bertani) agar; 10 g/L of tryptophan, 5 g/L of yeast extract, 10 g/L of NaCl, 15 g/L of agar) was inoculated into 30 ml of LB medium and cultured at 37°C for 12 hours. This seed culture was again inoculated into 3 L of LB medium containing 1 mM manganese sulfate (MnSO₄), and cultured at 37°C for 20 hours.

### Example 2.2. Isolation and purification of SodA2

The cell culture obtained in Example 2.1 was centrifuged at 4°C and 3,578 × g for 20 minutes to collect the supernatant, which was then concentrated 10-fold using ultrafiltration (hereinafter referred to as UF, MWCO 10,000). 390 g of ammonium sulfate was added thereto per liter of the concentrated cell culture, the mixture was stirred for 20 minutes, and centrifugation was performed to take the supernatant. The taken supernatant was purified using a phenyl Sepharose HP column. The purification process was as follows. The column was equilibrated using 50 mM potassium phosphate pH 7.0 with a concentration of 2 M ammonium sulfate. Then, the supernatant obtained by adding ammonium sulfate was passed through the column, and SodA2 attached to the column was recovered using 50 mM potassium phosphate pH 7.0 containing 1.6 M ammonium sulfate. The solution purified through the column was collected and concentrated by ultrafiltration while removing highly concentrated salts formed during the purification process. The concentrate was filtered through a sterilization filter and then lyophilized. Activity of SodA2 was analyzed using the SOD assay kit (Cayman Chemical, Michigan, USA). One unit of SOD activity is defined as an amount of the enzyme that inhibits superoxide radicals by 50%. Activity of the dried SodA2 enzyme ranged from 1000 to 1200 U/mg.

### Example 3. Preparation of substance to be administered

Shellac (EXCELACS Co., Ltd., Bangkok) was dissolved in ethanol to a concentration of 3% and sterilized using a 0.2 µm sterile filter. The shellac dissolved in ethanol was diluted with PBS. Lyophilized SodA2 was dissolved at 20 mg/mL, and the shellac solution and SOD solution were mixed in a 1:1 ratio, and then lyophilized. The substance used in the animal test was prepared by mixing the lyophilized shellac-coated SodA2 with dextrin in a ratio of 1:9 to 12 (shellac-coated SodA2: dextrin). The final substance was adjusted to have SOD activity of 90 to 110 U/mg.

### Example 4. Production of diabetic retinopathy model

One hundred twenty 6-week-old male C57BL/6 mice were purchased (from KOATECH) and maintained under a 12-h light/dark cycle, with free access to food and water. Diabetes mellitus (DM) mice were induced by intraperitoneal injection of streptozotocin (STZ) solution (10 mM citrate buffer (pH 4.5), 180 mg/kg). Three days after injection, blood glucose levels were measured, and only the mice with a blood glucose level of 300 mg/dl or higher were selected as diabetic mice. The C57BL/6 mice were classified into 10 groups, as shown in Table 6, depending on the concentration of the orally-administered drug and whether diabetes was induced, and the experiment was conducted by dividing the mice into a 3-week experimental group (60 mice) and a 16-week experimental group (60 mice) depending on the period of SodA2 administration. For the SodA2-administered group, SodA2 was administered daily starting from the streptozotocin injection stage regardless of the presence of diabetes.

**[Table 6]**

| | 3-week experimental group (total 60 mice) | 16-week experimental group (total 60 mice) |
|---|---|---|
| Normal control group | C57BL/6 control group administered with vehicle, n=6 | |
| Normal mouse group administered with 1 unit of SodA2 | C57BL/6 control group + 1 unit of SodA2, orally administered at 150 ul once a day, n=6 | |
| Normal mouse group administered with 5 units of SodA2 | C57BL/6 control group + 5 units of SodA2, orally administered at 150 ul once a day, n=6 | |
| Normal mouse group administered with 10 units of SodA2 | C57BL/6 control group + 10 units of SodA2, orally administered at 150 ul once a day, n=6 | |
| Normal mouse group administered with 20 units of SodA2 | C57BL/6 control group + 20 units of SodA2, orally administered at 150 ul once a day, n=6 | |
| Diabetic control group | C57BL/6 mice injected with STZ (DM), n=6 | |
| Diabetic group administered with 1 unit of SodA2 | C57BL/6 mice injected with STZ (DM) + 1 unit of SodA2, orally administered at 150 ul once a day, n=6 | |
| Diabetic group administered with 5 units of SodA2 | C57BL/6 mice injected with STZ (DM) + 5 units of SodA2, orally administered at 150 ul once a day, n=6 | |
| Diabetic group administered with 10 units of SodA2 | C57BL/6 mice injected with STZ (DM) + 10 units of SodA2, orally administered at 150 ul once a day, n=6 | |
| Diabetic group administered with 20 units of SodA2 | C57BL/6 mice injected with STZ (DM) + 20 units of SodA2, orally administered at 150 ul once a day, n=6 | |

### Example 5. Production of uveitis model

One hundred twenty 6-week-old male C57BL/6 mice were purchased (from KOATECH) and maintained on a 12-hour light/dark cycle, with free access to food and water. Of the total 120 mice, 60 mice were used for the endotoxin-induced uveitis model group and 60 mice were used for the experimental autoimmune uveitis model group. All animal experiments were performed in accordance with the statement of the Association for Research in Vision and Ophthalmology for the Use of Animals in Ophthalmic and Vision Research and were approved by the Institutional Animal Care and Use Committees of Seoul National University and Seoul National University Hospital.

### Example 5.1. Production of endotoxin-induced uveitis model and administration of SodA2

To produce an endotoxin-induced uveitis (EIU) model, the SodA2 drug was administered orally at concentrations of 1, 5, 10, and 20 units for 7 days (200 µl, once a day), and then acute uveitis was induced by intravitreal injection of 1 ng of LPS (lipopolysaccharide, L2880-10MG) that is a component of the outer membrane of gram-negative bacteria. For the mouse model used for the acute uveitis experiment, see Table 7.

**[Table 7]**

| Endotoxin-induced uveitis model (EIU, oral administration for 1 week) | |
|---|---|
| Normal control group | C57BL/6 control group administered with vehicle, n=6 |
| Normal mouse group administered with 1 unit of SodA2 | C57BL/6 control group + 1 unit of SodA2, orally administered at 150 ul once a day, n=6 |
| Normal mouse group administered with 5 units of SodA2 | C57BL/6 control group + 5 units of SodA2, orally administered at 150 ul once a day, n=6 |
| Normal mouse group administered with 10 units of SodA2 | C57BL/6 control group + 10 units of SodA2, orally administered at 150 ul once a day, n=6 |
| Normal mouse group administered with 20 units of SodA2 | C57BL/6 control group + 20 units of SodA2, orally administered at 150 ul once a day, n=6 |
| EIU control group | C57BL/6 mice with uveitis induced by LPS administration, n=6 |
| EIU mouse group administered with 1 unit of SodA2 | C57BL/6 mice with uveitis induced by LPS administration + 1 unit of SodA2, orally administered at 150 ul once a day, n=6 |
| EIU mouse group administered with units of SodA2 | C57BL/6 mice with uveitis induced by LPS administration + 5 units of SodA2, orally administered |
| | at 150 ul once a day, n=6 |
| EIU mouse group administered with 10 units of SodA2 | C57BL/6 mice with uveitis induced by LPS administration + 10 units of SodA2, orally administered at 150 ul once a day, n=6 |
| EIU mouse group administered with 20 units of SodA2 | C57BL/6 mice with uveitis induced by LPS administration + 20 units of SodA2, orally administered at 150 ul once a day, n=6 |

### Example 5.2. Production of experimental autoimmune uveitis model and administration of SodA2

The procedure for inducing T cell-mediated chronic uveitis to produce an experimental autoimmune uveitis (EAU) model was as follows. 30 mg of human IRBP₁₋₂₀ (Peptron, GPTHLFQPSLVLDMAKVLLD 95%) (Peptron) was dissolved in PBS and incubated at 26°C for 30 minutes. Next, mycobacterium tuberculosis H37 Ra (BD, 231141) was mixed with complete Freund's adjuvant (CFA; Sigma, F5881) and incubated at 26°C for 30 minutes, and then mixed with human IRBP₁₋₂₀ at a 1:1 ratio. A total of 200 ul of the mixed reagent was injected subcutaneously into two sites, which are the back of the neck and the hind legs, of C57BL/6 mice, and 200 ul of the pertussis toxin (sigma, P7208) was injected intraperitoneally to induce chronic uveitis. Next, the SodA2 drug was administered orally at concentrations of 1, 5, 10, and 20 units for 21 days (200 ul, once a day). For the mouse model used for the chronic uveitis experiment, see Table 8.

**[Table 8]**

| Experimental autoimmune uveitis model (EAU, oral administration for 3 weeks) | |
|---|---|
| Normal control group | C57BL/6 control group administered with vehicle, n=6 |
| Normal mouse group administered with 1 unit of SodA2 | C57BL/6 control group + 1 unit of SodA2, orally administered at 150 ul once a day, n=6 |
| Normal mouse group administered with 5 units of SodA2 | C57BL/6 control group + 5 units of SodA2, orally administered at 150 ul once a day, n=6 |
| Normal mouse group administered with 10 units of SodA2 | C57BL/6 control group + 10 units of SodA2, orally administered at 150 ul once a day, n=6 |
| Normal mouse group administered with 20 units of SodA2 | C57BL/6 control group + 20 units of SodA2, orally administered at 150 ul once a day, n=6 |
| EAU control group | C57BL/6 mice with autoimmune uveitis induced (EAU), n=6 |
| EAU mouse group administered with 1 unit of SodA2 | C57BL/6 mice with autoimmune uveitis induced (EAU) + 1 unit of SodA2, orally administered at 150 ul once a day, n=6 |
| EAU mouse group administered with 5 units of SodA2 | C57BL/6 mice with autoimmune uveitis induced (EAU) + 5 units of SodA2, orally administered at 150 ul once a day, n=6 |
| EAU mouse group administered with 10 units of SodA2 | C57BL/6 mice with autoimmune uveitis induced (EAU) + 10 units of SodA2, orally administered at 150 ul once a day, n=6 |
| EAU mouse group administered with 20 units of SodA2 | C57BL/6 mice with autoimmune uveitis induced (EAU) +20 units of SodA2, orally administered at 150 ul once a day, n=6 |

### Example 6. Identification of efficacy in diabetic retinopathy model

### Example 6.1. Evaluation of retinal vascular leakage

To determine the degree of vascular leakage in the 3-week and 16-week experimental groups after the end of the SodA2 administration period, the mice were anesthetized, intravenously injected with Evans blue (20 mg/ml, 150 µl, dissolved in PBS), and perfusion was performed for 1 hour. Then, the eyes were excised. All eye excision procedures were performed in accordance with the statement of the Association for Research in Vision and Ophthalmology for the Use of Animals in Ophthalmic and Vision Research. For qualitative analysis of retinal vascular leakage, the excised eyes were fixed in 4% paraformaldehyde (PFA), and then subjected to flat mounting in 1x PBS to be placed on a slide. The entire retina was imaged at 40x magnification using a fluorescence microscope (Eclipse 90i, Nikon, Tokyo, Japan). In addition, for quantitative analysis of retinal vascular leakage, the entire retina was imaged with red fluorescence. The images were adjusted to match the color threshold based on the automatic ISODATA algorithm using the ImageJ (1.47v, NIH, Bethesda, MD, USA) program. Then, the red areas were measured and expressed as the degree of retinal vascular leakage (%), and compared with the non-diabetic normal control group.

### 3-week administration group

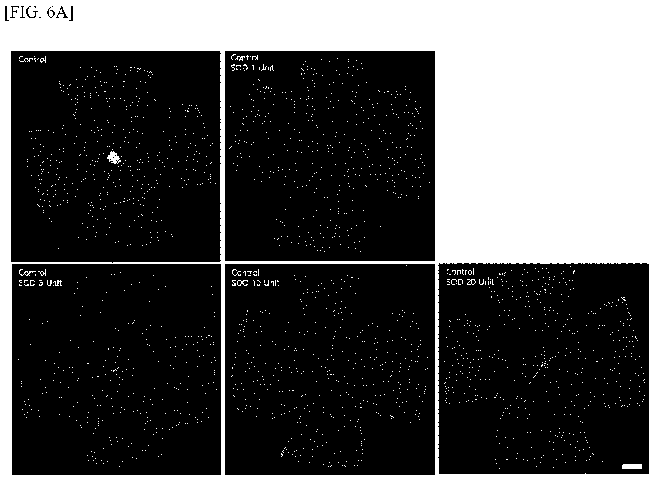

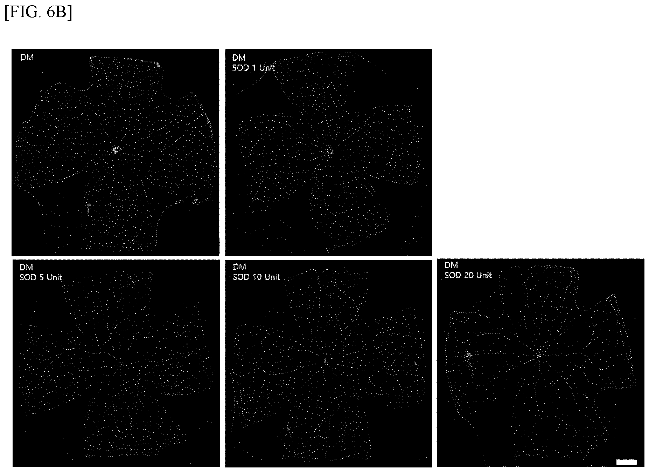

As a result of qualitative analysis using a microscope, no vascular leakage was observed in both the normal control group not administered with SodA2 and the normal mouse group administered with SodA2 (SodA2 concentrations of 1, 5, 10, and 20 units), and it was confirmed that there was no change in normal retinal vascular permeability regardless of SodA2 administration (FIG. 6A). For the diabetic control group, contrast enhancement within the retinal tissue due to increased vascular permeability was observed 3 weeks after induction of diabetes (the first image in FIG. 6B). On the other hand, for the diabetic group administered with SodA2, a significant decrease in retinal vascular permeability was observed compared to the control group (the second to fifth images in FIG. 6B).

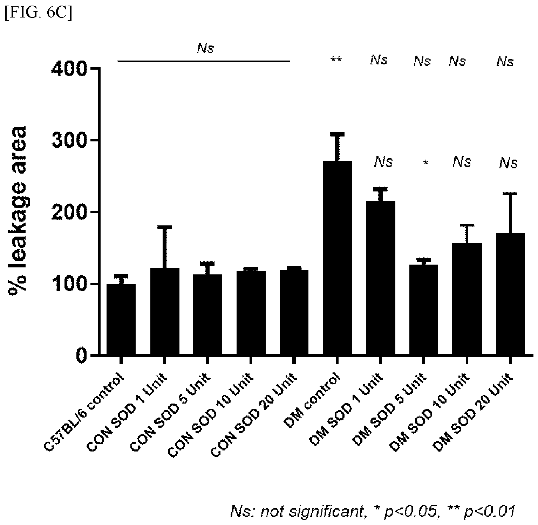

As a result of quantitative analysis of retinal vascular leakage, consistent with the qualitative evaluation, no retinal vascular leakage was observed in both the normal control group not administered with SodA2 and the normal mouse group administered with SodA2, and high retinal vascular leakage was observed in the diabetic control group. For the diabetic-induced 3-week experimental group administered with SodA2, a pattern of decreased retinal vascular permeability was observed compared to the diabetic control group, and in particular, a statistically significant decrease was observed in the diabetic group administered with 5 units of SodA2. The quantitative evaluation of retinal vascular leakage for the 3-week experimental group is shown in FIG. 6C.

### 16-week administration group

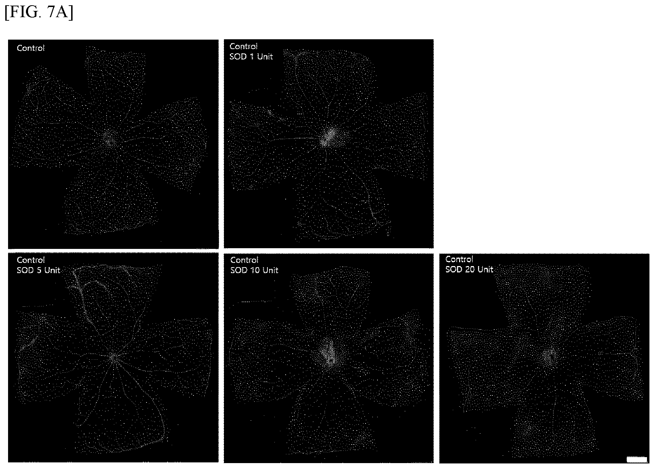

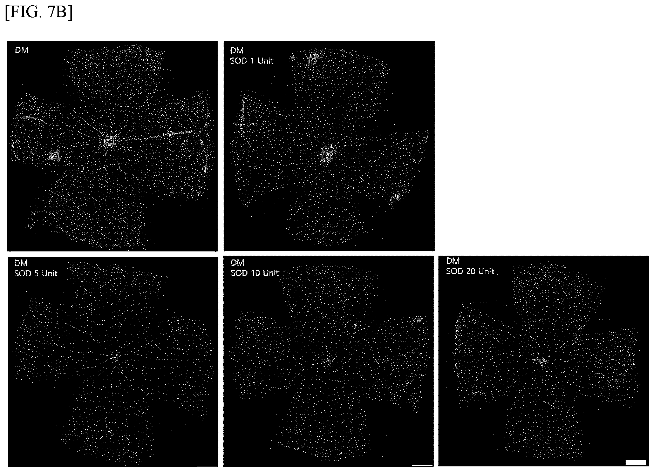

As a result of qualitative analysis using a microscope, no vascular leakage was observed in both the normal control group not administered with SodA2 and the normal mouse group administered with SodA2 (FIG. 7A). In some of the normal mouse groups administered with 10 and 20 units of SodA2, a pattern of increased retinal vascular permeability was observed compared to the non-diabetic control group, although it was not significant. For the diabetic control group, contrast enhancement within the retinal tissue due to increased vascular permeability was observed 16 weeks after induction of diabetes (the first image in FIG. 7B). On the other hand, for the diabetic group administered with SodA2, decreased retinal vascular permeability was observed compared to the control group (the second to fifth images in FIG. 7B), and in particular, vascular permeability was significantly decreased in the diabetic group administered with 5 units of SodA2.

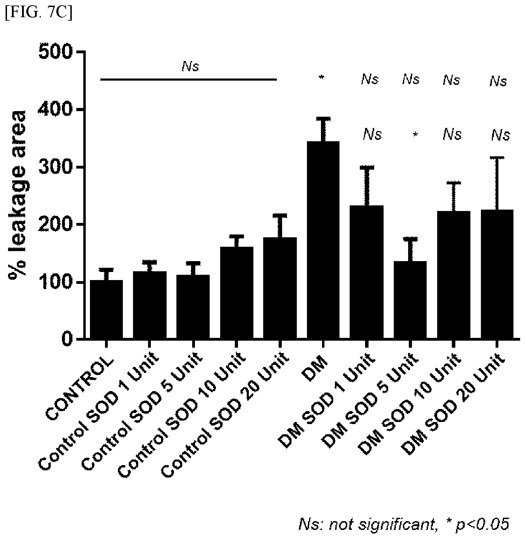

As a result of quantitative analysis of retinal vascular leakage, consistent with the qualitative evaluation, no retinal vascular leakage was observed in both the normal control group not administered with SodA2 and the normal mouse group administered with SodA2 (although increased retinal vascular permeability was observed in the normal mouse group administered with 20 units of SodA2, it was not significant), and increased retinal vascular leakage was observed in the diabetic control group. On the other hand, for the diabetic-induced 16-week group administered with SodA2, a pattern of decreased retinal vascular permeability was observed compared to the diabetic control group, and in particular, a statistically significant decrease was observed in the diabetic group administered with 5 units of SodA2. The quantitative evaluation of retinal vascular leakage for the 16-week experimental group is shown in FIG. 7C.

### Example 6.2. Evaluation of astrocyte immunofluorescence staining

The retina was isolated from the eyes fixed in 4% paraformaldehyde, and then incubated overnight at 4°C in Perm/Block solution (PBS solution containing 0.3% Triton-X and 0.2% BSA) with anti-GFAP antibody (1:100; Invitrogen, 53-9392-82) and isolectin B4-594 (1:100; Invitrogen, I21412). The next day, the retina was washed with PBS, and then mounted with fluoromount aqueous mounting medium (Sigma-Aldrich). To check distribution of astrocyte foot processes outside the pericytes surrounding the retinal vasculature (better distribution of astrocyte foot processes is interpreted as better inhibition of vascular permeability), the retina was stained and imaged with a confocal microscope (Leica TCS STED, Leica Microsystems, Wetzlar, Germany). The central part of the retina was imaged at × 400 magnification, and astrocytes were quantified by groups using the ImageJ (1.47v, NIH, Bethesda, MD, USA) program.

### 3-week administration group

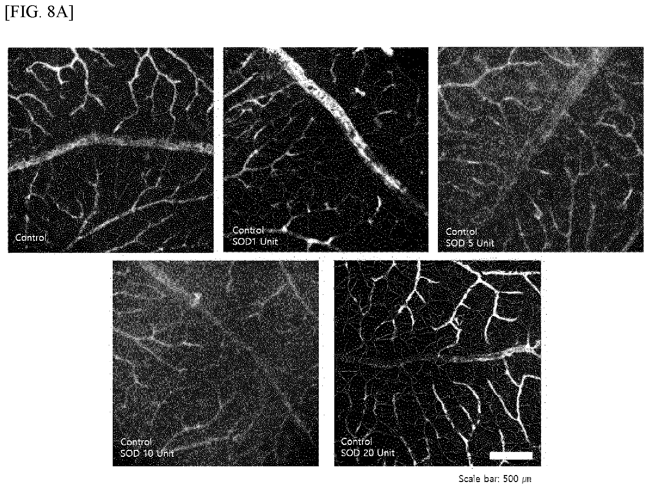

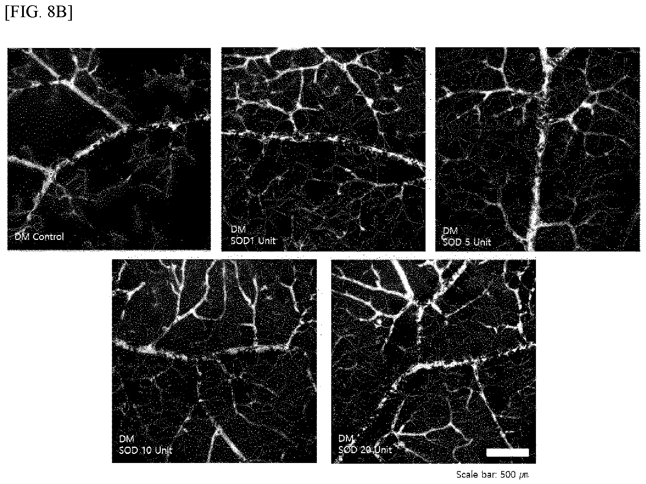

As a result of qualitative analysis using a microscope, there was no change in distribution of astrocyte foot processes in both the normal control group not administered with SodA2 and the normal mouse group administered with SodA2 (SodA2 concentrations of 1, 5, 10, and 20 units), regardless of the administration of SodA2 (FIG. 8A). For the diabetic control group, a decrease in astrocyte foot processes was observed 3 weeks after induction of diabetes, compared to the normal control group (the first image in FIG. 8B). On the other hand, for the diabetic group administered with SodA2, it was observed that the distribution of astrocyte foot processes was significantly increased compared to the control group, indicating recovery (the second to fifth images in FIG. 8B).

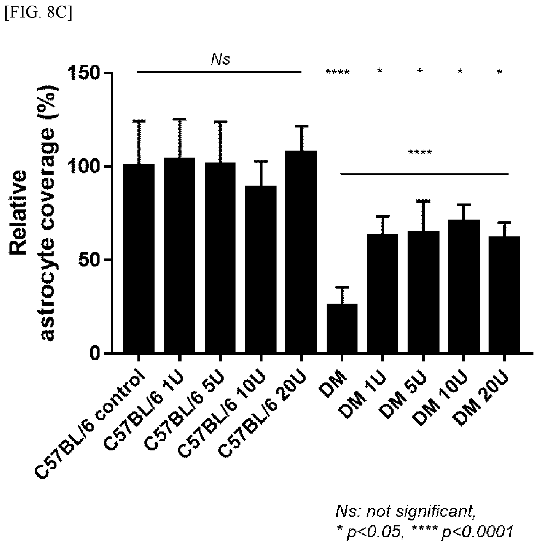

As a result of quantitative analysis of astrocyte immunofluorescence staining, consistent with the qualitative evaluation, no change was observed in the distribution of astrocyte foot processes in both the normal control group not administered with SodA2 and the normal mouse group administered with SodA2. For the diabetic control group, astrocyte foot processes were decreased 3 weeks after induction of diabetes. For the diabetic-induced experimental group administered with SodA2 for 3 weeks, distribution of astrocyte foot processes was increased, compared to the diabetic control group that showed a decrease in astrocyte foot processes. The quantitative evaluation of astrocyte immunofluorescence staining for the 3-week experimental group is shown in FIG. 8C.

### 16-week administration group

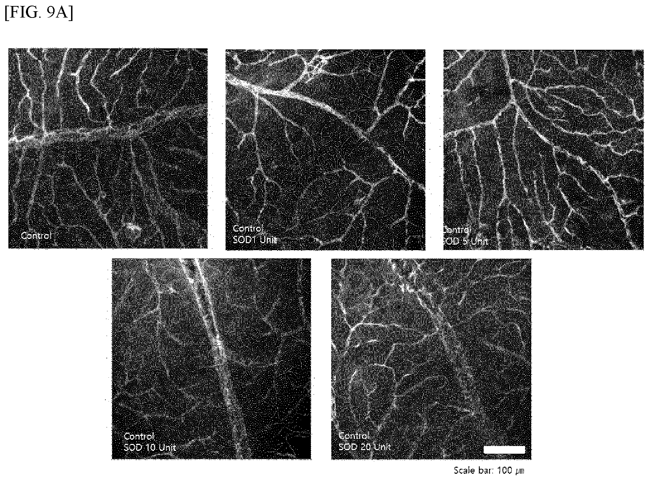

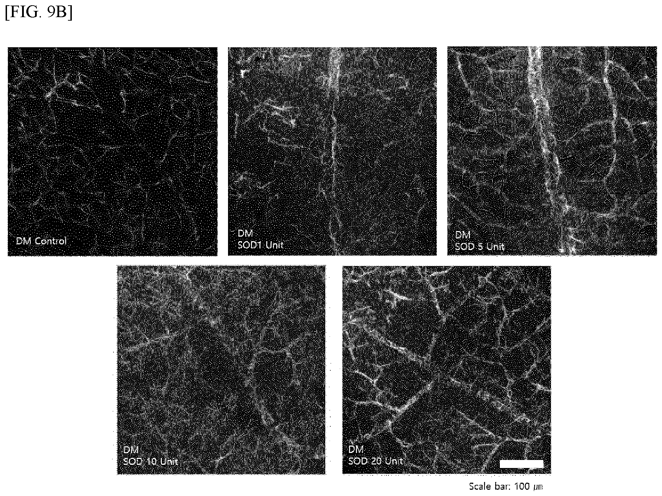

As a result of qualitative analysis using a microscope, unlike the normal control group not administered with SodA2, a pattern of decreased distribution in astrocyte foot processes was observed for some of the normal mouse group administered with SodA2, although it was not significant (FIG. 9A). For the diabetic control group, a significant decrease in astrocyte foot processes was observed 16 weeks after induction of diabetes, compared to the normal control group (the first image in FIG. 9B). For the diabetic group administered with SodA2, it was observed that the distribution of astrocyte foot processes was significantly increased compared to the control group, indicating recovery (the second to fifth images in FIG. 9B).

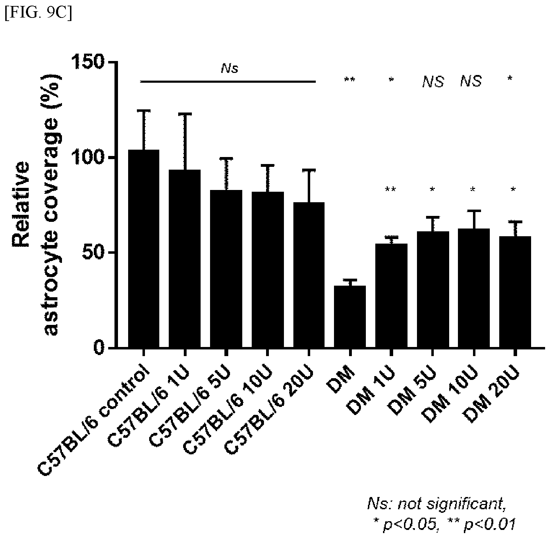

Quantitative analysis of astrocyte immunofluorescence staining showed the same pattern as the qualitative analysis. For the diabetic-induced 16-week experimental group administered with SodA2, a significant increase in the distribution of astrocyte foot processes was observed compared to the diabetic control group that showed decreased astrocyte foot processes after 16 weeks of diabetes induction. The quantitative evaluation of astrocyte immunofluorescence staining for the 16-week experimental group is shown in FIG. 9C.

### Example 6.3. Evaluation of retinal vascular staining

The retina was isolated from the eyes fixed in 4% paraformaldehyde, placed in a 24-well plate, and washed four times with PBS. Next, 3% trypsin solution (Difco 1:250, 0.1 M Tris (pH 7.8)) was added to the plate containing the retina and incubated at 37°C for 2 hours. After the reaction was completed, trypsin was carefully removed, and washing was performed with water. The resultant was transferred to a slide, and non-vascular tissues were carefully removed with water. The slide containing only blood vessels was dried, and then H&E staining was performed.

Diabetic retinopathy may lead to pericyte loss around blood vessels with acellular capillary formation, which was confirmed by retinal vascular staining.

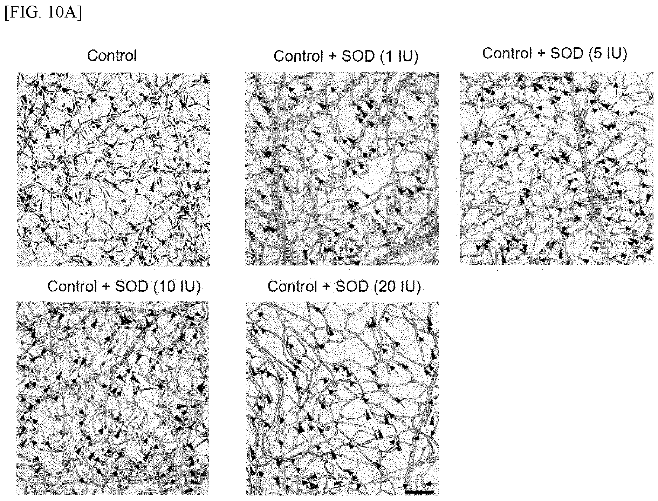

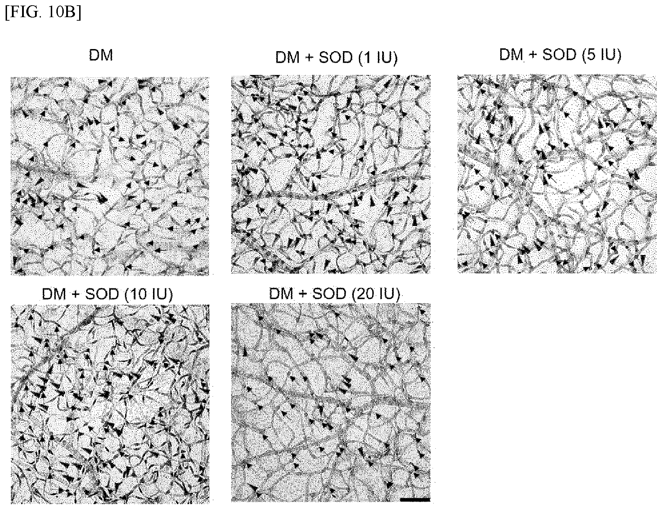

As a result of qualitative analysis using a microscope, loss of pericytes (indicated by arrows) or formation of acellular capillaries (indicated by arrowheads) were not observed in the 16-week normal control group not administered with SodA2 and the 16-week normal mouse group administered with SodA2 (FIG. 10A). For the diabetic control group, loss of pericytes and formation of acellular capillaries were increased 16 weeks after induction of diabetes (the first image in FIG. 10B). On the other hand, for the diabetic group administered with SodA2, inhibited pericyte loss and inhibited acellular capillary formation were observed (the second to fifth images in FIG. 10B).

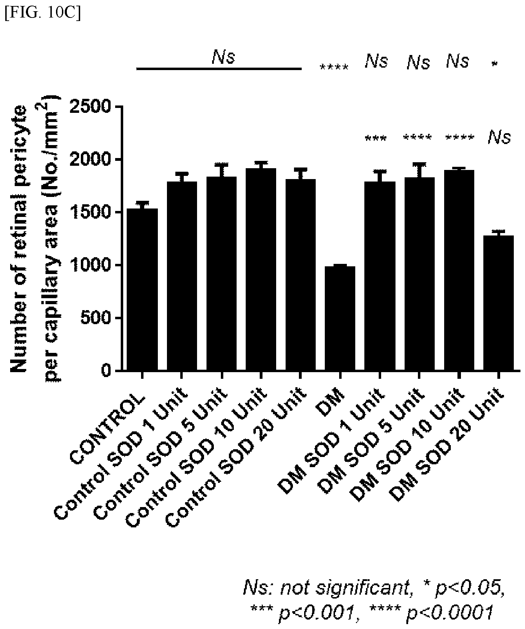

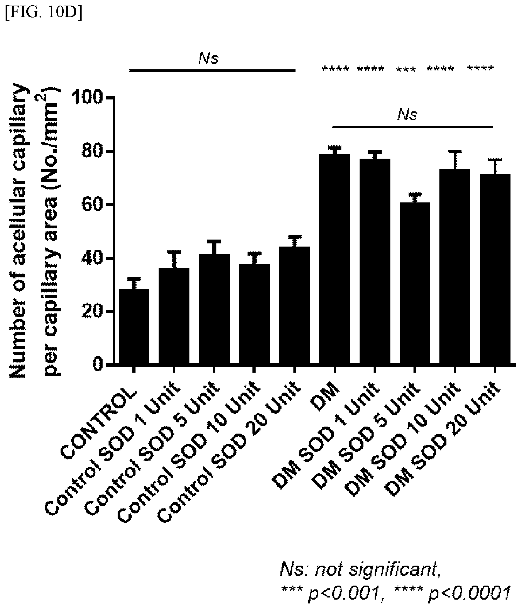

As a result of additional quantitative analysis, there were no changes in the number of pericytes and the number of acellular capillaries in both the normal control group not administered with SodA2 and the normal mouse group administered with SodA2 (the first to fifth columns from the left in FIGS. 10C and 10D). 16 weeks after induction of diabetes, a decrease in the number of pericytes (increased pericyte loss) was observed in the diabetic control group, whereas a significant increase in the number of pericytes was observed in the group administered with SodA2 for 16 weeks (the sixth to tenth columns from the left in FIG. 10C). In addition, 16 weeks after induction of diabetes, for the diabetic group administered with SodA2, in particular, the group administered with 5 units of SodA2, a reduced pattern of acellular capillary formation was observed, although it was not statistically significant (FIG. 10D).

### Example 7. Evaluation of experiments on ocular disease in uveitis model

### Example 7.1. Fundus evaluation of uveitis

For the endotoxin-induced uveitis (EIU) model, fundus examination was performed 24 hours after LPS injection (acute induction); and for the experimental autoimmune uveitis (EAU) model, fundus examination was performed on days 14, 16, 18, and 21 after uveitis induction (chronic induction). The fundus evaluation of uveitis was scored from 0 points, which indicate no intraocular inflammation, to 4 points, based on criteria such as hemorrhage or ocular condition. For the scoring criteria, see Table 9.

**[Table 9]**

| Fundus evaluation criteria of uveitis | |
|---|---|
| Score | Criteria |
| 4 points | Vitreous hemorrhage filling the vitreous cavity or large retinal detachment (1/2 or more) |
| 3.5 points | Vitreous hemorrhage (1/2 or more) or small retinal detachment |
| 3 points | Small amount of vitreous hemorrhage, pattern of linear lesion, large confluent lesions, or papilledema |
| 2.5 points | chorioretinal lesions (>5) or linear lesions (2 or more, 1/4 area) |
| 2 points | Small focal lesions (3 to 5) or linear lesions (1 or less, 1/2 or more area) |
| 1.5 points | Small focal lesions (3 to 5) or linear lesions (1 or less, 1/4 area) |
| 1 point | Small focal lesions (1 to 2) or minimal vasculitis/vitritis (1/2 or more area) |
| 0.5 points | Small focal lesions (1 to 2) or minimal vasculitis/vitritis (1/4 area) |
| 0 points | No inflammation |

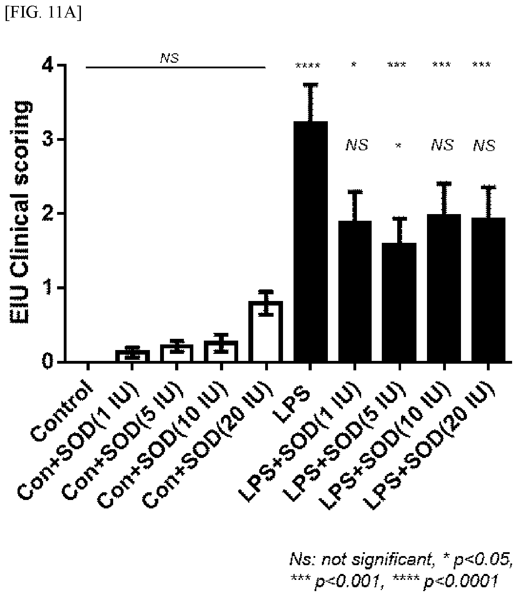

When evaluated according to the above-described evaluation criteria, for the EIU model (acute induced group), administration of SodA2 showed a significant decrease effect, compared to no-administration group (FIG. 11A).

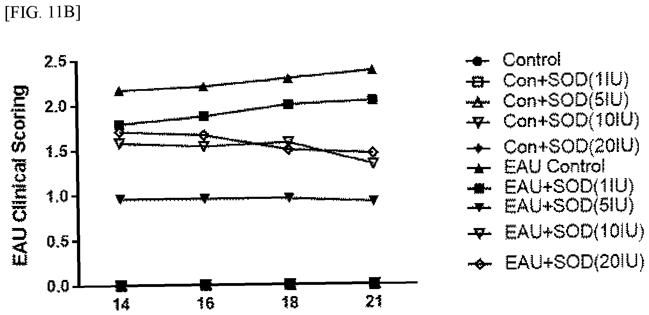

For EAU model (chronic induced group), administration of SodA2 showed a decrease effect in the fundus evaluation scoring of uveitis, compared to the no-administration group (FIG. 11B). In particular, inflammation was most significantly improved in the 5-unit SodA2-administered group.

### Example 7.2. H&E staining and tissue evaluation

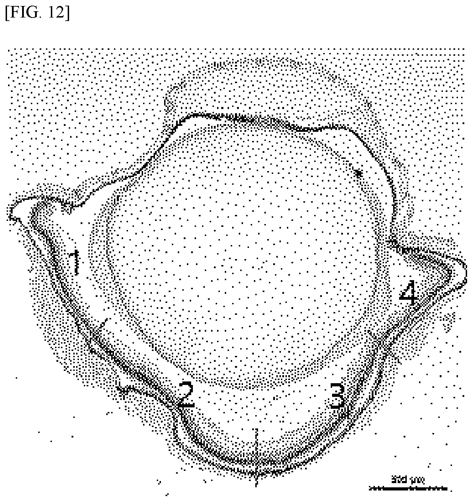

For tissue evaluation of uveitis, the excised eyes were fixed in 4% paraformaldehyde and embedded in paraffin blocks. The paraffin blocks were cut into 4 µm-thick sections and H&E (hematoxylin & eosin) staining was performed. For information on the tissue evaluation criteria of uveitis, see Table 10 and FIG. 12.

**[Table 10]**

| Tissue evaluation criteria of uveitis | |
|---|---|
| Score | Criteria |
| 4 points | Retinal fold (4 areas), intraretinal/subretinal hemorrhage or exudate (4 areas), damage to inner/outer retinal layers (4 areas), inflammatory cell infiltration (4 areas) |
| 3.5 points | Retinal fold (3 areas), intraretinal/subretinal hemorrhage or exudate (3 areas), damage to inner/outer retinal layers (3 areas), inflammatory cell infiltration (3 areas) |
| 3 points | Retinal fold (3 areas), intraretinal/subretinal hemorrhage or exudate (2 areas), damage to inner/outer retinal layers (2 areas), inflammatory cell infiltration (3 areas) |
| 2.5 points | Retinal fold (2 areas), intraretinal hemorrhage or exudate (2 areas), retinal inner layer damage (2 areas), inflammatory cell infiltration (2 areas) |
| 2 points | Retinal fold (2 areas), intraretinal hemorrhage or exudate (1 area), retinal inner layer damage (1 area), inflammatory cell infiltration (2 areas) |
| 1.5 points | Retinal fold (1 area), inflammatory cell infiltration (1 area), retinal inner layer damage (1 area) |
| 1 point | Retinal fold (1 area), inflammatory cell infiltration (1 area) |
| 0.5 points | Inflammatory cell infiltration (1 area), no retinal fold, no retinal hemorrhage or exudate, no retinal damage |
| 0 points | Normal retina |

### EIU model (acute induced group)

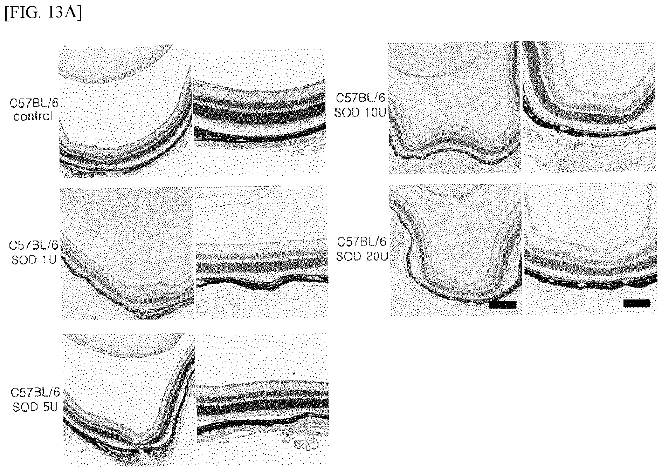

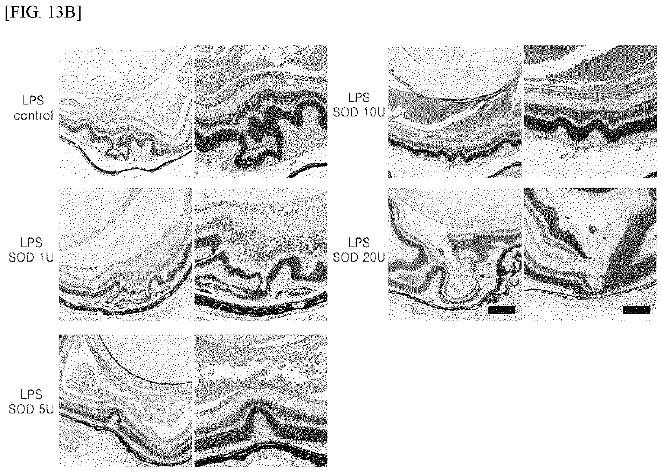

No histological abnormalities were observed in both the normal control group not administered with SodA2 and the normal mouse group administered with SodA2 (SodA2 concentrations of 1, 5, 10, and 20 units) (FIG. 13A). For the EIU control group induced by LPS injection, severe inflammation was observed in the vitreous body and retinal tissue (the first image in FIG. 13B). In contrast, for the EIU mouse group administered with SodA2, reduced inflammation in the vitreous body and reduced infiltration of inflammatory cells in the retinal tissue were observed, compared to the EIU control group (the second to fifth images in FIG. 13B).

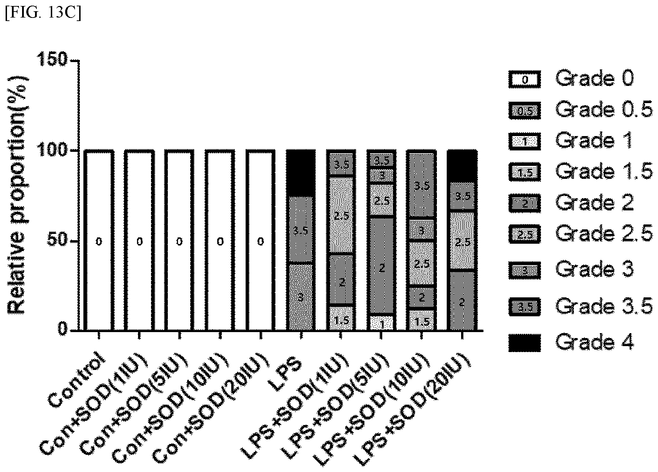

As a result of histological grading, it was identified that for the EIU mouse group administered with SodA2, histological inflammation was improved to a lower grade, compared to the EIU control group (FIG. 13C).

### EAU model (chronic induced group)

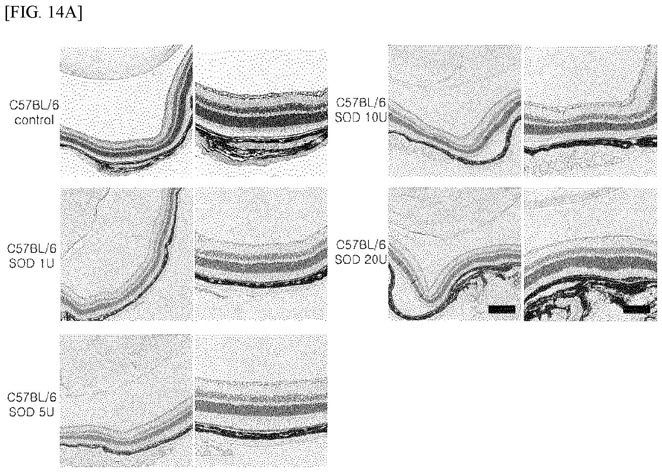

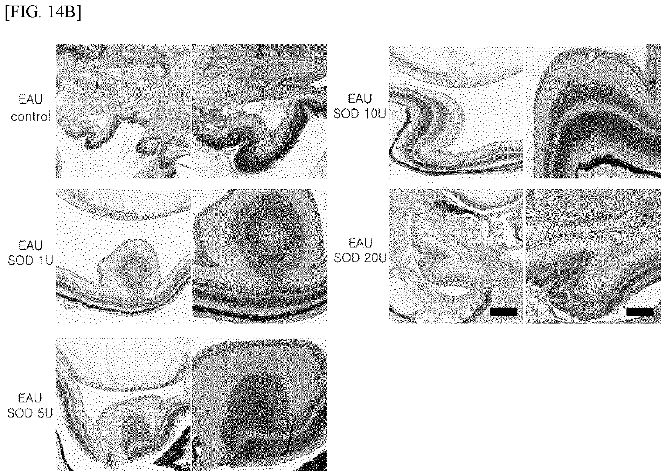

No histological abnormalities were observed in both the normal control group not administered with SodA2 and the normal mouse group administered with SodA2 (SodA2 concentrations of 1, 5, 10, and 20 units) (FIG. 14A). For the EAU control group in which uveitis was induced by autoimmune disease, infiltration of inflammatory cells into the retinal tissue and resulting retinal changes were observed (the first image in FIG. 14B). On the other hand, for the EAU mouse group administered with SodA2, infiltration of inflammatory cells into the retinal tissue and resulting retinal changes were significantly decreased compared to the EAU control group, and in particular, a remarkable decrease was observed in the 5- and 10-unit groups (the second to fourth images in FIG. 14B).

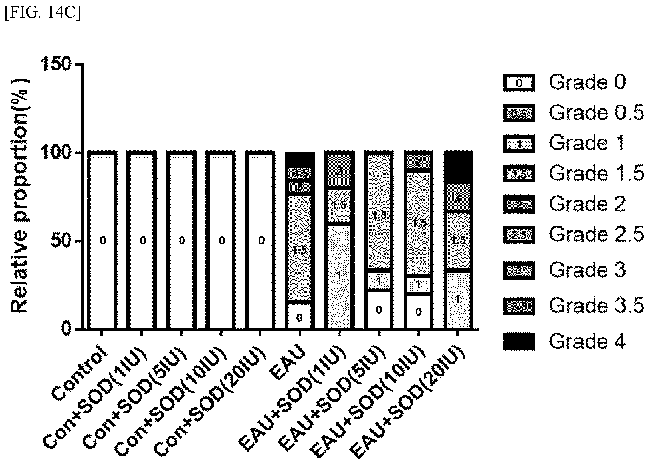

As a result of histological grading, similar to the tissue evaluation, it was identified that for the EAU mouse group administered with SodA2, histological inflammation was improved to a lower grade, compared to the EAU control group, and in particular, the 5- and 10-unit SodA2-administered EAU mouse groups showed improvement to a lower grade (FIG. 14C).

### Statistical processing

Statistics were performed using one-way ANOVA and Tukey's multiple comparisons test. GraphPad Prism 7.0 was used for statistical processing.

## Claims

1. A pharmaceutical composition for treating or preventing diabetic retinopathy or uveitis, comprising a superoxide dismutase (SOD) as an active ingredient.

2. The pharmaceutical composition of claim 1, wherein the diabetic retinopathy or uveitis comprises at least one symptom selected from the group consisting of vision impairment, ocular vascular leakage, and ocular inflammation.

3. The pharmaceutical composition of claim 1, wherein the SOD induces at least one effect selected from reduced vascular permeability, recovery of astrocyte foot process, inhibited pericyte loss, and inhibited acellular capillary formation in diabetic retinopathy.

4. The pharmaceutical composition of claim 1, wherein the SOD induces the following:
(i) an improvement or alleviation in at least one fundus evaluation indicator selected from focal lesion, linear lesion, chorioretinal lesion, confluent lesion, vasculitis, vitritis, vitreous hemorrhage, papilledema, and retinal detachment in uveitis;
(ii) an improvement or alleviation in at least one tissue evaluation indicator selected from inflammatory cell infiltration, retinal fold, intra/subretinal hemorrhage, intra/subretinal exudate, and retinal damage; or
(iii) both (i) and (ii).

5. The pharmaceutical composition of claim 1, wherein the uveitis is caused by an infectious or non-infectious factor.

6. The pharmaceutical composition of claim 5, wherein the infectious factor of the uveitis comprises at least one selected from bacteria, viruses, fungi, and parasites.

7. The pharmaceutical composition of claim 5, wherein the non-infectious factor of the uveitis comprises at least one selected from autoimmune diseases, tumor, trauma, surgery, and systemic diseases.

8. The pharmaceutical composition of claim 1, wherein the SOD is an isolated or purified enzyme.

9. The pharmaceutical composition of claim 1, wherein the SOD is included in a form of a strain lysate, a strain culture, a strain culture concentrate, a strain culture extract, or a dried form thereof.

10. The pharmaceutical composition of claim 1, wherein the SOD is a Mn-SOD.

11. The pharmaceutical composition of claim 1, wherein the SOD is a deamidated Mn-SOD.

12. The pharmaceutical composition of claim 1, wherein the SOD is derived from a Bacillus species strain.

13. The pharmaceutical composition of claim 1, wherein the SOD is derived from *Bacillus amyloliquefaciens* GF423 strain (KCTC 13222BP).

14. The pharmaceutical composition of claim 1, wherein amino acid residues 73 and 136 of the SOD are substituted with Asp residues, with respect to SEQ ID NO: 2.

15. The pharmaceutical composition of claim 1, wherein the SOD comprises an amino acid sequence as set forth in SEQ ID NO: 4.

16. The pharmaceutical composition of claim 1, wherein the composition is for oral administration.

17. The pharmaceutical composition of claim 16, wherein the SOD is coated with a coating agent.

18. A veterinary composition for treating or preventing diabetic retinopathy or uveitis, comprising a superoxide dismutase (SOD) as an active ingredient.

19. A food composition for ameliorating or preventing diabetic retinopathy or uveitis, comprising a superoxide dismutase (SOD) as an active ingredient.

20. A feed composition for ameliorating or preventing diabetic retinopathy or uveitis, comprising a superoxide dismutase (SOD) as an active ingredient.

21. A method for treating or preventing diabetic retinopathy or uveitis, comprising administering to a subject the pharmaceutical composition of any one of claims 1 to 17 or a superoxide dismutase (SOD).

22. A use of the pharmaceutical composition of any one of claims 1 to 17 or a superoxide dismutase (SOD), for preventing or treating diabetic retinopathy or uveitis.

23. A use of the pharmaceutical composition of any one of claims 1 to 17 or a superoxide dismutase (SOD), in manufacture of a medicament for prevention or treatment of diabetic retinopathy or uveitis.
